# EUROPEAN PATENT APPLICATION

(11) **EP 3 117 756 A1**
(43) Date of publication of application: **18.01.2017**
(21) Application number: 14885494.6
(22) Date of filing: 16.12.2014
(51) Int. Cl.: A61B 1/00, G02B 23/24

(54) **ENDOSCOPE AND ENDOSCOPE SYSTEM**

(30) Priority: 12.03.2014 JP 2014048870
(71) Applicant: Olympus Corporation, Tokyo 192-8507 (JP)
(72) Inventor: TAKEMOTO Shotaro, Tokyo 192-8507 (JP); BANJU Kazuo, Tokyo 192-8507 (JP)
(74) Representative: Schicker, Silvia
(86) International application number: PCT/JP2014/083310
(87) International publication number: WO 2015/136807

(57) **Abstract**

An endoscope includes an insertion part; an operation part attached to a proximal end part of the insertion part; a shaft-like member inserted into the insertion part, a proximal end part of the shaft-like member extends to the operation part, and the shaft-like member configured to be capable of advancing and retracting with respect to the insertion part; and a projectable unit provided at a distal end part of the insertion part; wherein the projectable unit includes a fixing part watertightly attached on an outer surface of the insertion part; a movable part provided in a state where a distal end is exposed to an outside, and is movable in a direction intersecting the outer surface by advancing and retracting the shaft-like member; and a coupling part which is elastically deformable and watertightly couples together the fixing part and the movable part.

## Description

### Technical Field

The present invention relates to an endoscope for inserting into and performing treatment to a patient's body or the like, and an endoscope system including the endoscope.

Priority is claimed on Japanese Patent Application No. 2014-48870, filed on March 12, 2014, the content of which is incorporated herein by reference.

### Background Art

In the related art, various kinds of treatment is performed by inserting a treatment tool into the inside of a body through a channel formed in an endoscope while observing the inside of a patient's body with the endoscope.

For example, an endoscope described in Patent Document 1 has an insertion part, an operation part, and a connector.

The insertion part is an elongated region that is inserted into an inspection region, such as an inside of a body cavity, and has a distal end part, an angle part, and a flexible part.

A CCD sensor, an optical system for imaging the inspection region using the CCD sensor, and a processing substrate that processes an output signal of the CCD sensor are arranged in the distal end part.

In the endoscope configured in this way, forceps are inserted into the inspection region through a channel in a procedure to perform sampling of tissue.

### Prior Art Document

### Patent Document

[Patent Document 1] Japanese Unexamined Patent Application, First Publication No. 2009-165640

### Disclosure of the Invention

### Problem to be solved by the Invention

However, such an endoscope is not a disposable device and is used repeatedly. For this reason, it is necessary to perform cleaning by washing the inside of the channel with a brush or the like and sterilizing with a drug solution after being used in a procedure. The cleaning of the channel needs to pass the brush through a conduit line over its entire length. Moreover, since it is necessary to sterilize the inside of the channel reliably, a large amount of labor is required.

The invention has been made in view of such problems, and an object thereof is to provide an endoscope in which the time and effort for cleaning the inside of a channel after being used in a procedure is reduced, and an endoscope system using the endoscope.

### Means for Solving the Problems

An endoscope according to a first aspect of the invention includes: an insertion part; an operation part attached to a proximal end part of the insertion part; a shaft-like member inserted into the insertion part, a proximal end part of the shaft-like member extending to the operation part, and the shaft-like member being configured to be capable of advancing and retracting with respect to the insertion part; and a projectable unit provided at a distal end part of the insertion part; wherein the projectable unit includes: a fixing part watertightly attached on an outer surface of the insertion part; a movable part provided in a state where a distal end is exposed to an outside, and is movable in a direction intersecting the outer surface by advancing and retracting the shaft-like member; and a coupling part which is elastically deformable and watertightly couples together the fixing part and the movable part.

According to a second aspect of the present invention, in the endoscope according to the first aspect, the endoscope may further include a distal-end-side restricting part restricting a range where the shaft-like member moves to a distal end side with respect to the insertion part.

According to a third aspect of the present invention, in the endoscope according to the first aspect, the endoscope may further include a a proximal-end-side restricting part restricting a range where the shaft-like member moves to a proximal side with respect to the insertion part.

According to a fourth aspect of the present invention, in the endoscope according to the first aspect, the shaft-like member and the movable part may be separated from each other when the shaft-like member moves to a proximal side, and wherein the movable part may be configured to be pushed to a distal end side by the shaft-like member when the shaft-like member moves to the distal end side.

According to a fifth aspect of the present invention, in the endoscope according to the fourth aspect, a proximal end of the movable part may be formed so as to project toward proximally than the coupling part, one of a proximal end part of the movable part and a distal end of the shaft-like member may be provided with a connecting part, and the other of the proximal end part of the movable part and the distal end of the shaft-like member may be provided with a larger-diameter part that is larger than the external diameter of the connecting part.

According to a sixth aspect of the present invention, in the endoscope according to the fifth aspect, a recess that is recessed toward a direction away from the connecting part may be formed in a surface of the larger-diameter part that faces the connecting part.

According to a seventh aspect of the present invention, in the endoscope according to the first aspect, the shaft-like member and the movable part may be coupled together.

According to an eighth aspect of the present invention includes the endoscope according to the first aspect; and a cap that is attachable to and detachable from an insertion part distal end that is an outer surface of the insertion part of the endoscope. The cap includes: a main body that is formed in a tubular shape; a proximal end part of the main body attached to the insertion part; and a movable member that is movably attached to the main body and that moves in a direction intersecting a central axis of the main body, and moves to a position distally side than the main body, when being moved from a state where the main body is attached to the insertion part and the movable part is moved from the insertion part distal end to the proximal end side, to a state where the movable part is moved from the insertion part distal end to the distal end side.

### Effects of the Invention

According to the above endoscope and the above endoscope system, the time and effort for cleaning the inside of a channel after being used in a procedure is reduced.

### Brief Description of Drawings

FIG. 1 is an overall view of an endoscope according to a first embodiment of the invention.
FIG. 2 is a cross-sectional view of a distal end part of an insertion part of the endoscope according to the first embodiment of the invention.
FIG. 3 is a cross-sectional view of main parts of an operation part of the endoscope according to the first embodiment of the invention.
FIG. 4 is a cross-sectional view of the distal end part of the insertion part when a driving wire of the endoscope according to the first embodiment of the invention is pushed in.
FIG. 5 is a view showing the action of the endoscope according to the first embodiment of the invention.
FIG. 6 is a cross-sectional view of the distal end part of the insertion part in the endoscope of a modified example of the first embodiment of the invention.
FIG. 7 is a cross-sectional view of main parts in the endoscope of a modified example of the first embodiment of the invention.
FIG. 8 is a cross-sectional view of the main parts in the endoscope of a modified example of the first embodiment of the invention.
FIG. 9 is a cross-sectional view of the main parts in the endoscope of a modified example of the first embodiment of the invention.
FIG. 10 is a cross-sectional view of the main parts in the endoscope of a modified example of the first embodiment of the invention.
FIG. 11 is a cross-sectional view of the main parts in the endoscope of a modified example of the first embodiment of the invention.
FIG. 12 is a cross-sectional view of the main parts when the driving wire of the endoscope of the modified example of the first embodiment of the invention is pushed in.
FIG. 13 is an exploded perspective view of a distal end part of an endoscope system according to a second embodiment of the invention.
FIG. 14 is a front view of the distal end part of the endoscope system according to the second embodiment of the invention.
FIG. 15 is a cross-sectional view of main parts showing the behavior of the endoscope system according to the second embodiment of the invention.
FIG. 16 is a cross-sectional view of the main parts showing the behavior of the endoscope system according to the second embodiment of the invention.
FIG. 17 is a perspective view of an attachment of a modified example of the second embodiment of the invention.
FIG. 18 is a front view of a distal end part when the attachment shown in FIG. 17 is attached to the insertion part of the endoscope.
FIG. 19 is a front view of the distal end part of the endoscope system of a modified example of the second embodiment of the invention.
FIG. 20 is a perspective view of an attachment of the endoscope system of the modified example of the second embodiment of the invention is used by cutting away a part of the attachment.

### Description of Embodiments

### (First Embodiment)

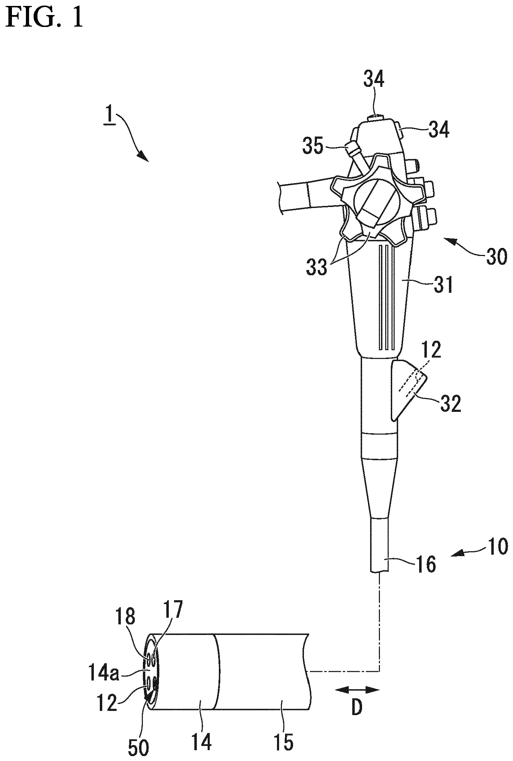

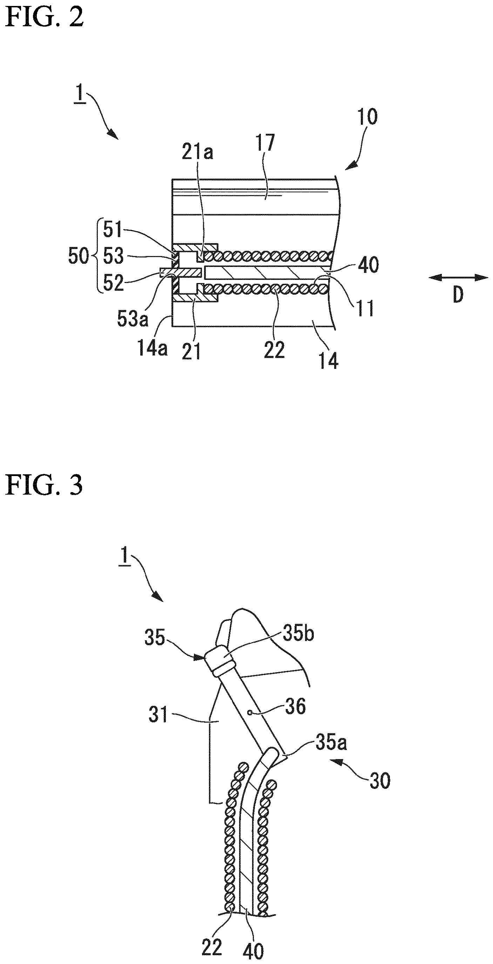

Hereinafter, a first embodiment of an endoscope system according to the invention will be described, referring to FIGS. 1 to 12. FIG. 1 is an overall view of an endoscope 1 according to the first embodiment of the invention. FIG. 2 is a cross-sectional view of a distal end part of an insertion part 10 of the endoscope 1 according to the first embodiment of the invention.

As shown in FIGS. 1 and 2, the endoscope 1 according to the present embodiment is a so-called flexible endoscope, and includes an elongated insertion part 10, an operation part 30, a driving wire (shaft-like member) 40, and a projectable unit 50. The operation part 30 is attached to a proximal end part of the insertion part 10. The driving wire 40 is inserted into a first channel (channel) 11 formed in the insertion part 10. The projectable unit 50 is provided at the distal end part of the insertion part 10.

As shown in FIG. 1, the insertion part 10 is formed in a columnar shape as a whole, and has a distal end rigid part 14, a bending part 15, and a flexible tube part 16. The distal end rigid part 14 is provided on a distal end side of the insertion part 10. The bending part 15 is attached to a proximal end side of the distal end rigid part 14, and is configured to be bendably operated. The flexible tube part 16 is attached to the proximal end side of the bending part 15.

A distal end part of a light guide 17 and an imaging unit 18 having a CCD (not shown) are provided on a distal end surface (outer surface) 14a of the distal end rigid part 14 in an externally exposed state.

The aforementioned first channel 11 and a second channel 12 are formed in the insertion part 10, and distal end parts of the channels 11 and 12 open to the distal end surface 14a of the distal end rigid part 14. The channels 11 and 12 extend in a longitudinal direction D of the insertion part 10, and proximal end parts of the channels 11 and 12 extend to the operation part 30.

As shown in FIG. 2, the first channel 11 is constituted with a cylindrical mouthpiece 21 provided on the distal end surface 14a, and an inner peripheral surface of a tube 22 connected to the proximal end side of the mouthpiece 21.

A diameter-reduced part 21a that projects from an inner peripheral surface of the mouthpiece 21 is formed over the whole circumference of the mouthpiece 21 in the mouthpiece 21. The mouthpiece 21 and the diameter-reduced part 21a are integrally formed of metal, such as stainless steel.

The tube 22 is formed of a multiple-strand coil. In addition to this, the tube 22 may be formed of, for example, a densely-wound coil, a tube made of resin, or the like. A distal end part of the tube 22 is fixed to a stepped part formed at a connection portion between the mouthpiece 21 and the diameter-reduced part 21a with an adhesive, welding, or the like.

Although not shown, the second channel 12 consists of an inner peripheral surface of a tube, such as a coil.

The driving wire 40 is formed of a material having flexibility, such as a metallic single wire or a metallic stranded wire. The driving wire 40 is inserted into the first channel 11 so as to be capable of advancing and retracting in the longitudinal direction D with respect to the insertion part 10. The external diameter of the driving wire 40 is slightly smaller than the internal diameter of the tube 22 and the internal diameter of the diameter-reduced part 21a.

A proximal end part of the driving wire 40 extends to the operation part 30.

The aforementioned projectable unit 50 is attached to a distal end part of the mouthpiece 21. The projectable unit 50, as shown in FIG. 2, has a fixing part 51, a shaft (movable part) 52, and a coupling part 53. The fixing part 51 is watertightly attached to the distal end part of the mouthpiece 21. The shaft 52 is provided within the mouthpiece 21 in a state where a distal end thereof is exposed to the outside. The coupling part 53 watertightly couples together the fixing part 51 and the shaft 52.

The fixing part 51 and the coupling part 53 are integrally formed in the shape of a disk and formed of an elastically deformable material, such as silicone. A through-hole 53a penetrating in the longitudinal direction D is formed at a central part of the coupling part 53.

The shaft 52 is formed in the shape of a rod that extends in the longitudinal direction D, and formed of metal, such as stainless steel, or thermoplastic resin. The shaft 52 is harder than the coupling part 53. In the present embodiment, the distal end part of the shaft 52 projects to distally side than the coupling part 53, and the proximal end part of the shaft 52 projects to proximally side than the coupling part 53. In a natural state where an external force does not act on the shaft 52 and the coupling part 53, the distal end part of the shaft 52 projects to distally side than the distal end surface 14a of the distal end rigid part 14.

The mouthpiece 21 and the fixing part 51 are watertightly joined together with a well-known adhesive or the like. The through-hole 53a of the coupling part 53 and the shaft 52 are watertightly joined together with a well-known adhesive or the like.

The shaft 52 and the driving wire 40 are disposed on the same axis extending along the longitudinal direction D. Although the shaft 52 and the driving wire 40 can be separated from each other, the distal end part of the driving wire 40 comes into contact with the proximal end part of the shaft 52 when the driving wire 40 is moved (pushed in) to the distal end side with respect to the insertion part 10.

Although not shown, a plurality of bending pieces that are arranged side by side in the longitudinal direction D and are rockably connected to each other are built into the bending part 15. A distal end part of an operating wire is fixed to a bending piece being arranged closest to the distal end side among these bending pieces.

As shown in FIG. 1, a forceps port 32 is provided on a distal end side of an operation part main body 31 that constitutes the operation part 30. A proximal end part of the second channel 12 opens to the forceps port 32.

An angle knob 33 for operating the aforementioned operating wire, a light source (not shown), a monitor, a switch 34 for operating the aforementioned imaging unit 18 or the like, and a lever 35 for operating the driving wire 40 are provided on a proximal end side of the operation part main body 31.

The bending part 15 of the insertion part 10 can be bent in a desired direction by operating the angle knob 33.

The monitor and the imaging unit 18 are electrically connected together via an electric wire (not shown) inserted into the insertion part 10.

FIG. 3 is a cross-sectional view of main parts of the operation part 30. As shown in FIG. 3, the lever 35 is connected to the operation part main body 31 via a pin 36 provided at an intermediate part of the lever 35 in the longitudinal direction. A first end part 35a of the lever 35 is provided with a hole (not shown), and the proximal end part of the driving wire 40 is connected to the first end part 35a of the lever 35 so as to be rotatable in the circumferential direction of the hole in a state where the proximal end is inserted through this hole.

A proximal end part of the tube 22 extends to the vicinity of the first end part 35a of the lever 35.

In the endoscope 1 configured in this way, the lever 35 can be rotated around the pin 36 by a user operating a second end part 35b of the lever 35 with his/her finger or the like.

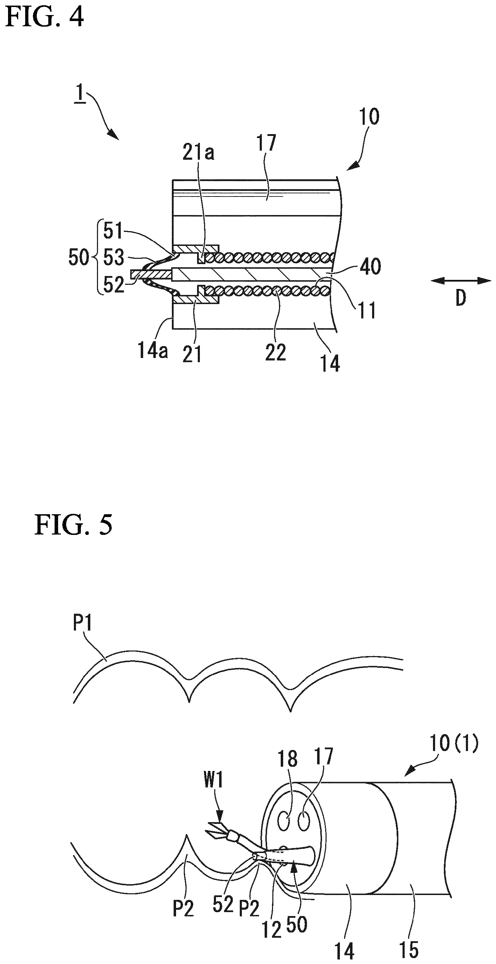

If the first end part 35a of the lever 35 is made to approach the proximal end part of the tube 22, as shown in FIG. 4, the driving wire 40 is moved and pushed into the distal end side with respect to the tube 22, and a distal end surface of the driving wire 40 comes into contact with a proximal end surface of the shaft 52. The distal end part of the driving wire 40 pushes the proximal end part of the shaft 52 toward the distal end side by further pushing the driving wire 40 and thereby the shaft 52 moves to the distal end side. As a result, the shaft 52 moves so as to be directed to the outside of the insertion part 10 from the distal end surface 14a of the insertion part 10 and projects to the distal end of the endoscope 1, and the coupling part 53 is deformed elastically. Hereinbelow, this state is referred to as a "projected state".

In the present embodiment, since the tube 22 is formed of a multiple-strand coil, the tube 22 is hardly elongate when the driving wire 40 is pushed in. Therefore, a length by which the driving wire 40 is pushed in with respect to the tube 22 on the proximal end side, and a length by which the driving wire 40 moves with respect to the tube 22 on the distal end side are approximately equal to each other. As a result, the shaft 52 of the projectable unit 50 can be stably operated by the driving wire 40.

The mouthpiece 21 functions as a positioning part that determines a position of the shaft 52 so that the distal end part of the driving wire 40 pushes the proximal end part of the shaft 52 when the driving wire 40 is pushed into the distal end side.

When the shaft 52 is brought into the projected state, a portion of the shaft 52 and a portion of the mouthpiece 21 may overlap each other in the longitudinal direction D. By adopting such a configuration, the shaft 52 in the projected state becomes hard together with the mouthpiece 21, i.e., the distal end rigid part 14, thereby the shaft 52 hardly tilts so as to incline with respect to the longitudinal direction D.

Meanwhile, if the first end part 35a of the lever 35 is separated from the proximal end part of the tube 22, as shown in FIG. 2, the driving wire 40 is moved (pulled back) to the proximal side with respect to the tube 22, and the coupling part 53 is deformed due to its own elastic force, thereby the fixing part 51 and the coupling part 53 return to the disk shape. The shaft 52 moves to the proximal side so as to be directed to the inside of the insertion part 10 from the distal end surface 14a of the insertion part 10 and is brought into a retracted state. As a result, the driving wire 40 and the shaft 52 are separated from each other.

By operating the lever 35 in this way, the shaft 52 can be moved in the longitudinal direction D, i.e., in a direction orthogonal to the distal end surface 14a.

Next, regarding the action of the endoscope 1 configured as described above will be described by using a case where a procedure is performed within the large intestine will be described as an example. FIG. 5 is a view showing the behavior of the endoscope 1.

The lever 35 is operated outside a patient's body, the shaft 52 is brought into the retracted state by pulling back the driving wire 40. The driving wire 40 and the shaft 52 are separated from each other.

The illumination light emitted from the light source is guided to the light guide 17 and illuminates the front of the insertion part 10 by operating the switch 34 of the operation part 30 to actuate the light source. An image in front of the insertion part 10 acquired by the imaging unit 18 is displayed on the monitor. The user inserts the insertion part 10 of the endoscope 1 into the large intestine P1 through a patient's anus, as shown in FIG. 5, while checking the image displayed on the monitor.

The insertion part 10 is inserted along the large intestine, and the angle knob 33 is operated to bend the bending part 15 if necessary. The position of the driving wire 40 in the longitudinal direction D with respect to the first channel 11 may move according to bend of the bending part 15 or the flexible tube part 16. Even in this case, since the driving wire 40 and the shaft 52 are separated from each other, the shaft 52 is prevented from being pushed against the driving wire 40.

Since the projectable unit 50 is watertightly attached to the distal end surface 14a of the insertion part 10, a body fluid or the like does not enter the first channel 11.

If the distal end side of the insertion part 10 is inserted into the large intestine P1, the second end part 35b of the lever 35 is operated, the driving wire 40 is pushed into the distal end side, and the shaft 52 is brought into the projected state. By bending the bending part 15, the distal end part of the shaft 52 is pushed against a fold P2 formed on the inner surface of the large intestine P1, and the height of the fold P2 is made low. In the imaging unit 18, a back side of the fold P2 that is made low is observed by the imaging unit 18. Although the back side of the fold P2 is not easily observed normally, observation is allowed by pushing the fold using the shaft 52 in this way.

If necessary, a treatment tool W1, such as forceps, is inserted into the large intestine P1 through the second channel 12 from the forceps port 32, and suitable treatment is performed.

As described above, according to the endoscope 1 according to the present embodiment, the projectable unit 50 is provided on the distal end surface 14a of the insertion part 10. By bringing the shaft 52 into the retracted state outside a patient's body and by inserting the insertion part 10 into a patient, a burden to be imposed on the patient at the time of insertion of the insertion part 10 can be reduced. When the insertion part 10 reaches a target region for observation or treatment, the shaft 52 is brought into the projected state, and the fold P2 or the like of the large intestine P1 is pushed by the shaft 52. Accordingly, the target region can be easily observed by the endoscope 1.

Since the projectable unit 50 is watertightly attached to the distal end surface 14a of the insertion part 10, a body fluid or the like does not enter the first channel 11. Therefore, the time and effort for cleaning the inside of the first channel 11 after the projectable unit is used for a procedure is reduced.

Since the coupling part 53 and the driving wire 40 do not come into direct contact with each other, the coupling part 53 can be prevented from wearing out, and the durability of the coupling part 53 can be improved.

The driving wire 40 and the shaft 52 are separated from each other when the driving wire 40 is pulled back, and the shaft 52 is pushed to the distal end side by the driving wire 40 when the driving wire 40 is pushed in.

Even in a case where the position of the driving wire 40 in the longitudinal direction D with respect to the first channel 11 moves, such as when the insertion part 10 is bent, a projection length of the shaft 52 that projects from the distal end surface 14a of the insertion part 10 to the distal end side when the driving wire 40 pushes the shaft 52 to the distal end side can be limited to increase.

The distal end of the driving wire 40 tends to move further to the proximal side with respect to the first channel 11 when the insertion part 10 is bent than when the insertion part 10 is straight. For this reason, it is possible to make an adjustment so that the driving wire 40 and the shaft 52 come into contact with each other or are slightly separated from each other when the insertion part 10 is straight. It is advantageous for durability that the driving wire 40 and the shaft 52 does not contact with each other beyond necessity.

Since the driving wire 40 has flexibility, the bending operation of the bending part 15 becomes easy, and the flexible tube part 16 can be easily bent.

For example, if the driving wire is arranged along an outer surface of the insertion part instead of the inside of the first channel, an entire external diameter of both the insertion part and the driving wire becomes large, and a sectional shape orthogonal to the longitudinal direction D does not easily become circular. For this reason, when whole of the insertion part and the driving wire are rotated around the longitudinal direction, the burden to be imposed on a patient increases.

However, in the endoscope 1 according to the present embodiment, since the driving wire 40 is inserted into the insertion part 10, the burden to be imposed on a patient when the insertion part 10 is rotated around the longitudinal direction D can be reduced.

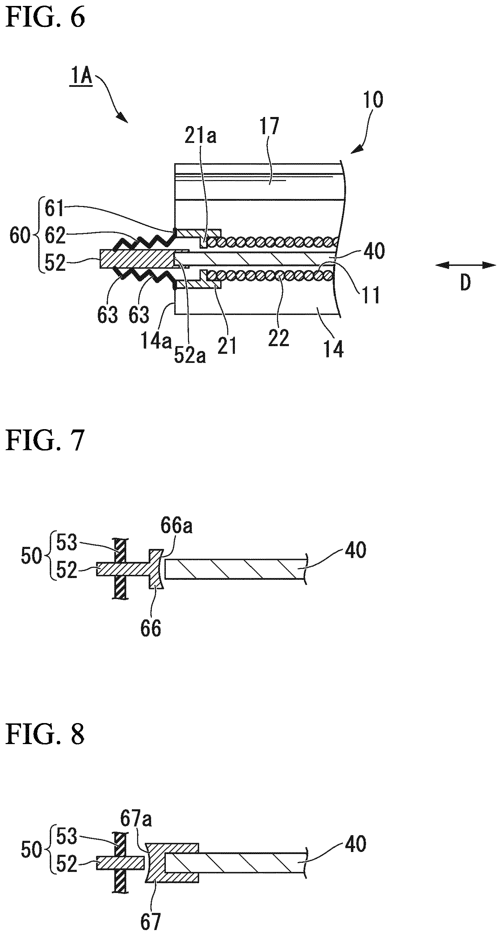

In the present embodiment, the configuration of the endoscope 1 can be modified as described below. FIGS. 6 to 11 are cross-sectional views of the distal end part of the insertion part in the endoscope of modified examples of the first embodiment of the invention.

As in an endoscope 1A of a modified example shown in FIG. 6, the fixing part 61 and the coupling part 62 of the projectable unit 60 may be integrally formed by a bellows-like member that has a plurality of fold parts 63 and is formed in a tubular shape.

The fixing part 61 and the coupling part 62 are formed of an elastically deformable material, such as resin. The fixing part 61 is arranged the proximally side than the coupling part 62, and is watertightly attached to the distal end part of the mouthpiece 21 with a well-known adhesive or the like. The shaft 52 is inserted into the coupling part 62, and a distal end part of the coupling part 62 and the shaft 52 are watertightly attached to each other.

The shaft 52 is in the retracted state when the respective fold parts 63 are brought into close contact with each other in the longitudinal direction D, and the shaft 52 is in the projected state when the respective fold parts 63 are stretched in the longitudinal direction D.

By adjusting the number of fold parts 63 of the coupling part 62, the projection length of the shaft 52 that projects from the distal end surface 14a of the insertion part 10 toward the distal end side when being brought into the projected state can be adjusted to a desired length.

Since the bellows-like member has good elasticity, the projectable unit 60 of the present modified example can increase the movement range of the shaft 52 in the longitudinal direction D.

In the present modified example, the distal end part of the driving wire 40 and the proximal end part of the shaft 52 are coupled together. A hole 52a is formed in the proximal end surface of the shaft 52. The distal end part of the driving wire 40 is fixed to the shaft 52 with an adhesive, brazing, soldering, or the like (not shown) in a state where the distal end part of the driving wire 40 is inserted into the hole 52a of the shaft 52.

According to the endoscope 1A of the modified example configured in this way, since the driving wire 40 and the shaft 52 are not separated from each other, a force that pulls back the driving wire 40 can be exerted on the shaft 52, and the movement range of the shaft 52 in the longitudinal direction D can be increased.

As in a modified example shown in FIG. 7, the proximal end part of the shaft 52 may be provided with a larger-diameter part 66 which is larger than the external diameter of the distal end part (connecting part) of the driving wire 40. A recess 66a, which is recessed in a hemispherical shape in a direction away from the distal end part of the driving wire 40, that is, toward the distal end side, may be formed in the surface of the larger-diameter part 66 that faces the distal end part of the driving wire 40. An external diameter of the recess 66a is greater than the external diameter of the driving wire 40. The larger-diameter part 66 is formed integrally with the shaft 52 with the same material as the shaft 52. In this modified example, the shaft 52 and the driving wire 40 are separable from each other.

By providing the larger-diameter part 66 in the shaft 52, even in a case where an axis on which the shaft 52 is arranged, and an axis on which the driving wire 40 is arranged are shifted from each other, a force that pushes the driving wire 40 can be easily transmitted to the shaft 52 via the larger-diameter part 66.

In the present modified example, since the recess 66a is formed in the larger-diameter part 66, the distal end part of the driving wire 40 being pushed in enters the recess 66a. Therefore, the driving wire 40 that has come into contact with the shaft 52 when the driving wire 40 is pushed in can be prevented from dropping out of the shaft 52.

A larger-diameter part 67 provided at the distal end part of the driving wire 40 shown in FIG. 8 may be included instead of the larger-diameter part 66 of the present modified example. The external diameter of the larger-diameter part 67 is larger than the external diameter of the proximal end part (connecting part) of the shaft 52. A recess 67a, which is recessed in a hemispherical shape in a direction away from the proximal end part of the shaft 52, i.e., toward the proximal side, may be formed in a surface of the larger-diameter part 67 that faces the proximal end part of the shaft 52.

Even if such a configuration is adopted, the same effects as the aforementioned modified example can be exhibited.

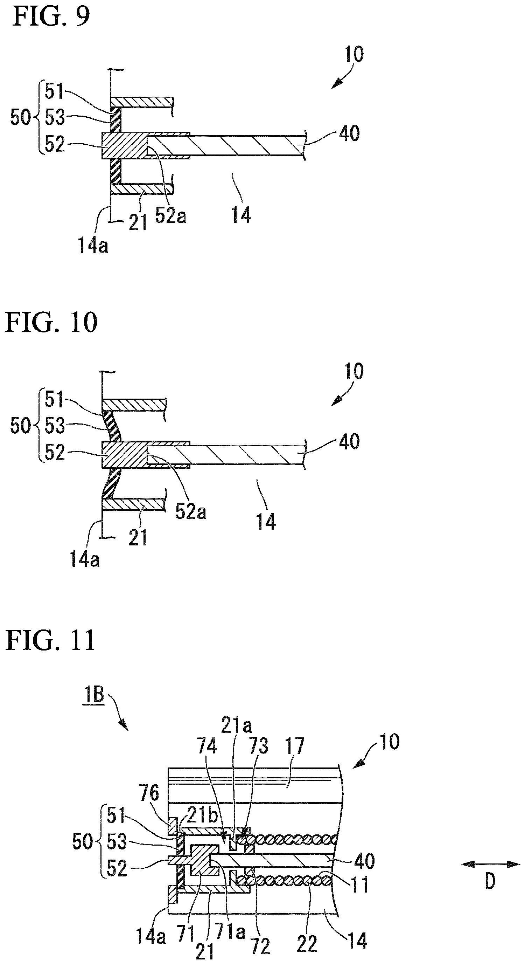

As in a modified example shown in FIG. 9, a configuration may be adopted in which the driving wire 40 and the shaft 52 are coupled together, and the distal end part of the shaft 52 projects further toward the distal end side than the distal end surface 14a of the distal end rigid part 14 when the driving wire 40 is pulled back and the shaft 52 is brought into the retracted state. In this modified example, the fixing part 51 and the coupling part 53 are formed in the shape of a flat disk in a natural state.

As in a modified example shown in FIG. 10, the distal end of part the shaft 52 may be configured so as not to project further toward the distal end side than the distal end surface 14a of the distal end rigid part 14 when the shaft 52 is brought into the retracted state. In this modified example, the fixing part 51 and the coupling part 53 are formed in the shape of a disk that is bent so that a central part thereof is recessed toward the proximal side in a natural state. By adopting such a configuration, since the distal end part of the shaft 52 does not project from the distal end surface 14a of the distal end rigid part 14 at the time of insertion of the insertion part 10, a burden to be imposed on a patient can be further reduced.

As in an endoscope 1B of a modified example shown in FIG. 11, a configuration may be adopted in which a diameter-enlarged part 71 provided at the proximal end part of the shaft 52 and the driving wire 40 are coupled together, and a stopper 72 is provided on the outer peripheral surface of the driving wire 40 positioned at the proximally side than the diameter-reduced part 21a of the mouthpiece 21.

The external diameter of the diameter-enlarged part 71 is larger than the internal diameter of the diameter-reduced part 21a. A hole 71a is formed in a proximal end surface of the diameter-enlarged part 71. The diameter-enlarged part 71 is formed integrally with the shaft 52 with the same material as the shaft 52.

The distal end part of the driving wire 40 is fixed to the diameter-enlarged part 71 with an adhesive (not shown) in a state where the distal end part of the driving wire 40 is inserted into the hole 71a of the diameter-enlarged part 71.

The stopper 72 is formed in the shape of a ring, using metal and thermoplastic resin, and is fixed to the driving wire 40. The external diameter of the stopper 72 is smaller than the internal diameter of the tube 22, and is larger than the internal diameter of the diameter-reduced part 21a. In this modified example, the fixing part 51 and the coupling part 53 are formed in the shape of a flat disk in a natural state.

The diameter-reduced part 21a and the stopper 72 constitute a distal-end-side restricting part 73, and the diameter-reduced part 21a and the diameter-enlarged part 71 constitute a proximal-end-side restricting part 74. It is preferable that the restricting parts 73 and 74 are provided in the vicinity of the distal end of the insertion part 10. The driving wire 40 elongates due to tensile stress and changes in its entire length, and the position of the distal end of the driving wire 40 with respect to the insertion part 10 changes depending on the shape of the insertion part 10. This is because the relative positions between the projectable unit 50 and the distal end of the driving wire 40 are shifted from each other even if the restricting parts 73 and 74 are provided on the proximal end side of the insertion part 10.

A stepped part 21b in which an internal diameter is enlarged is formed at the distal end part of the inner peripheral surface of the mouthpiece 21. The fixing part 51 of the projectable unit 50 is arranged within the stepped part 21b. A ring-shaped lid member 76 is arranged on a distal end surface of the mouthpiece 21. The internal diameter of the lid member 76 is approximately equal to an internal diameter of a portion that is located on the proximal end side of the mouthpiece 21 and is not provided with the diameter-reduced part 21a. The lid member 76 can be formed of the same material as the mouthpiece 21.

The fixing part 51 is watertightly attached to the mouthpiece 21 and the lid member 76 with a well-known adhesive or the like, in a state where the fixing part is sandwiched between the surface of the stepped part 21b on the proximal end side and the lid member 76 in the longitudinal direction D. The mouthpiece 21 and the lid member 76 may be fixed by a screw member.

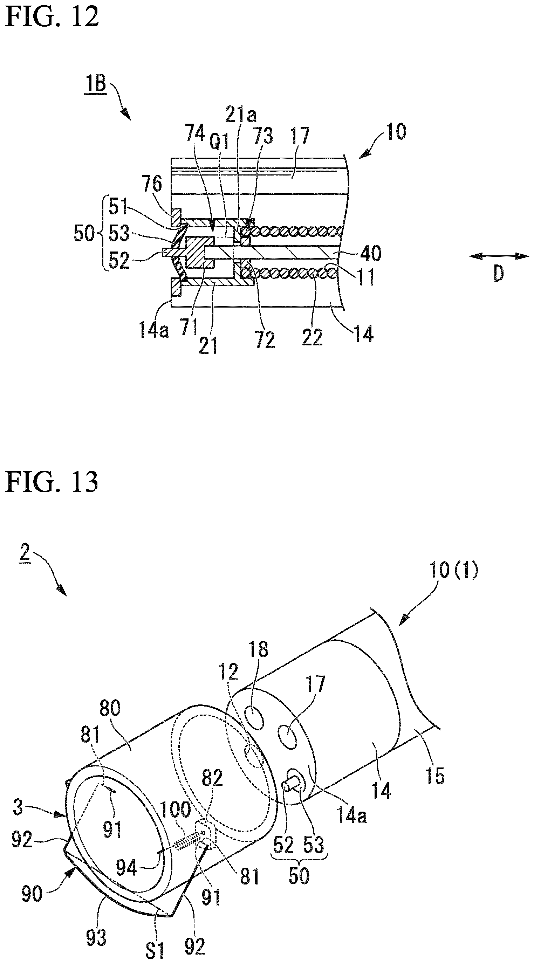

In the endoscope 1B configured in this way, if the driving wire 40 is pushed in from the state shown in FIG. 11, the stopper 72 abuts against the diameter-reduced part 21a as shown in FIG. 12, and the shaft 52 moves toward the distal end side and is brought into the projected state. Since the stopper 72 abuts against the diameter-reduced part 21a, the range where the driving wire 40 moves toward the distal end side with respect to the insertion part 10 is restricted.

Meanwhile, if the driving wire 40 is pulled back from the state shown in FIG. 11, the diameter-enlarged part 71 moves to a position Q1 shown in FIG. 12, and abuts against the diameter-reduced part 21a, and the shaft 52 moves to the proximal side and is brought into the retracted state. Since the diameter-enlarged part 71 abuts against the diameter-reduced part 21a, the range where the driving wire 40 moves to the proximal side with respect to the insertion part 10 is restricted.

Since the fixing part 51 is sandwiched between the stepped part 21b and the lid member 76, even if the shaft 52 moves in the longitudinal direction D, a gap between the distal end part of the mouthpiece 21 and the fixing part 51 is reliably held in a watertight state.

According to the endoscope 1B of the modified example configured in this way, since the distal-end-side restricting part 73 is included, the range where the driving wire 40 moves to the distal end side can be restricted, and an excessive force directed to the distal end side can be prevented from acting on the shaft 52 or the coupling part 53. Since the proximal-end-side restricting part 74 is included, the range where the driving wire 40 moves to the proximal side can be restricted, and an excessive force directed to the proximal side can be prevented from acting on the shaft 52 or the coupling part 53.

In the present modified example, a configuration may be adopted in which the endoscope 1B includes either the distal-end-side restricting part 73 or the proximal-end-side restricting part 74.

Although an example in which the second channel 12 is formed in the endoscope 1 is shown in the present embodiment, it is not necessary to include the second channel 12. In this case, in the aforementioned procedure, in addition to endoscope 1 according to the present embodiment, an insertion part of another endoscope may be inserted into the large intestine P1 and treatment may be performed, or treatment may be performed by a treatment tool inserted through a channel of this endoscope.

The first channel 11 may not be formed in the insertion part 10. In this case, the driving wire 40 together with the aforementioned electric wire or the like is inserted into the insertion part 10. It is preferable that the driving wire 40 is inserted through a coil within the insertion part 10.

The present embodiment has a configuration in which the distal end part of the shaft 52 projects to the distally side than the coupling part 53, and the proximal end part of the shaft 52 projects to the proximally side than the coupling part 53. However, the proximal end surface of the shaft 52 may be flush with the proximal end surface of the coupling part 53, or the distal end surface of the shaft 52 may be flush with the distal end surface of the coupling part 53.

In the present embodiment, a configuration may be adopted in which the proximal end part of the first channel 11 opens to the forceps port 32, and the lever 35 is rotatably supported in the forceps port 32 by the pin 36.

### (Second Embodiment)

Next, although a second embodiment of the invention will be described referring to FIGS. 13 to 20, the same parts as the above embodiment will be designated by the same reference signs and the description thereof will be omitted, and only different points will be described.

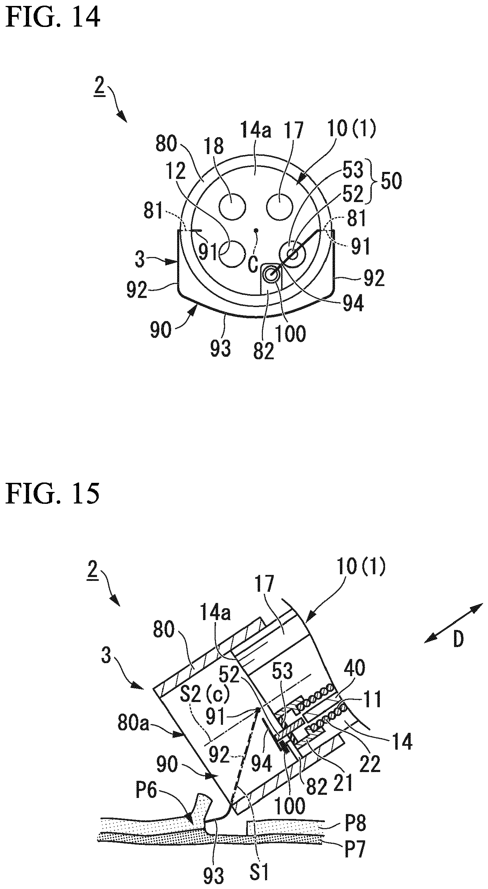

FIG. 13 is an exploded perspective view of a distal end part of an endoscope system 2 according to the present embodiment. FIG. 14 is a front view of the distal end part of the endoscope system 2 according to the present embodiment. As shown in FIGS. 13 and 14, the endoscope system 2 according to the present embodiment includes the endoscope 1 according to the first embodiment, and a detachable attachment (cap) 3 that is attachable to and detachable from the distal end surface 14a (insertion part distal end) of the insertion part 10 of the endoscope 1.

The attachment 3 has a main body 80 and a bar (movable member) 90. The main body 80 is formed in a cylindrical shape. The bar 90 is movably attached to the main body 80.

A pair of through-holes 81 is formed in an intermediate part of the main body 80 in the longitudinal direction. The pair of through-holes is formed at mutually facing positions so as to penetrate from an outer peripheral surface of the main body 80 to an inner peripheral surface thereof. A plate-like supporting member 82 that projects radially inward is provided on a proximal end side of the inner peripheral surface of the main body 80. The main body 80 and the supporting member 82 are integrally formed of resin having biocompatibility, such as polypropylene resin or polycarbonate resin.

The internal diameter of the main body 80 is slightly larger than an external diameter of the distal end rigid part 14 of the insertion part 10. For this reason, the main body 80 can be press-fitted into and fixed to the distal end rigid part 14.

As shown in FIG. 13, the bar 90 has a pair of shaft members 91, a pair of arm members 92, a coupling member 93, and a receiving member 94. The pair of shaft members 91 is respectively inserted into the through-holes 81 of the main body 80. The pair of arm members 92 extends from the portions of the respective shaft members 91 that project from the outer peripheral surface of the main body 80. The coupling member 93 is joined to the distal end parts of the respective arm members 92. The receiving member 94 extends from the portion of one shaft member 91 that projects from the inner peripheral surface of the main body 80.

Each arm member 92 extends in diagonally forward of the main body 80 and in a direction substantially orthogonal to the shaft member 91. The pair of shaft members 91 and the pair of arm members 92 are respectively arranged on a first reference plane S1. The coupling member 93 is formed in a curved shape that convex toward the distal end side on a plane intersecting the first reference plane S 1.

As shown in FIG. 15, the pair of arm members 92 and the receiving member 94 are arranged on the same side with respect to a second reference plane S2 that includes the axes of the pair of shaft members 91 and are parallel to the longitudinal direction D.

The pair of shaft members 91, the pair of arm members 92, the coupling member 93, and the receiving member 94 are integrally formed by bending a wire formed of stainless steel, titanium, or the like having biocompatibility and elasticity.

The bar 90 can rotate with respect to the main body 80 around the pair of through-holes 81 as a center by rotating each shaft member 91 within each through-hole 81 of the main body 80.

As shown in FIG. 15, the coupling member 93 is arranged at a position distally side than an edge of an opening 80a on the distal end side of the main body 80 in a natural state. Since the coupling member 93 is arranged so as to avoid a position distally side than a central part of the opening 80a of the main body 80 in a natural state, the coupling member is configured so as not to hinder the visual field of the imaging unit 18.

As shown in FIGS. 13 and 14, a spring member 100 is provided between the supporting member 82 and an end part of the receiving member 94 positioned opposite to a portion where is continued to the shaft member 91. A densely wound helical spring in a natural state is used for the spring member 100, and extends in an axis direction of the main body 80.

A distal end part of the spring member 100 is attached to the receiving member 94, and a proximal end part thereof is attached to the supporting member 82.

When the attachment 3 configured in this way is attached to the distal end surface 14a of the insertion part 10 in which the shaft 52 is brought into the retracted state, the attachment 3 is positioned as follows with respect to the distal end surface 14a of the insertion part 10.

That is, as shown in FIGS. 14 and 15, the insertion part 10 is inserted from an opening on the proximal end side of the main body 80 of the attachment 3. The attachment 3 is rotated around a central axis C of the main body 80 with respect to the insertion part 10, and the receiving member 94 is arranged distally side than the shaft 52 of the projectable unit 50. Accordingly, the position of the attachment 3 in the circumferential direction with respect to the insertion part 10 is specified (positioned).

The attachment 3 is pushed into the insertion part 10, and as shown in FIG. 15, the distal end surface 14a of the insertion part 10 is brought into contact with a proximal end surface of the supporting member 82. Accordingly, the position of the attachment 3 in the longitudinal direction D of the insertion part 10 with respect to the insertion part 10 is specified. In this case, the distal end part of the shaft 52 comes into contact with the outer surface of the receiving member 94 on the proximal end side.

Next, regarding the behavior of the endoscope system 2 configured as described above, a case where a procedure of mucosal resection within a body cavity is performed will be described as an example.

The insertion part 10 to which the attachment 3 is attached is inserted from a natural opening, such as a patient's mouth. The attachment 3 is inserted up to a lesioned mucous membrane portion P6 shown in FIG. 15 that is a target part to be resected.

Although not shown, a well-known injection needle may be inserted into the body cavity through the second channel 12, the injection needle may be made to project further toward the distal end side than the attachment 3, a physiological salt solution may be injected into a submucosal layer P7 of the lesioned mucous membrane portion P6, and the lesioned mucous membrane portion P6 may be upheaved. Thereafter, the injection needle is pulled out and extracted from the second channel 12.

A high-frequency knife is inserted into the body cavity through the second channel 12, and is made to project further toward the distal end side than the attachment 3. A portion of a mucous membrane P8 around the lesioned mucous membrane portion P6 is incised by the inserted high-frequency knife. Thereafter, the high-frequency knife is pulled back within the second channel 12.

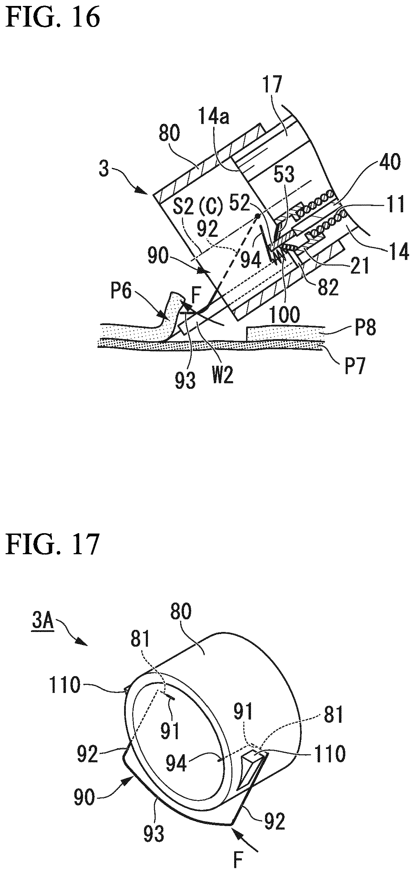

As shown in FIG. 15, the coupling member 93 of the bar 90 is inserted between the incised lesioned mucous membrane portion P6 and the submucosal layer P7. The lever 35 of the endoscope 1 is operated to bring the shaft 52 from the retracted state to the projected state. As shown in FIG. 16, since the shaft 52 pushes the receiving member 94 to the distal end side against the elastic force of the spring member 100, each shaft member 91 rotates within each through-hole 81 of the main body 80, and the pair of arm members 92 and the coupling member 93 rotate about the pair of through-holes 81 as a center. In this case, the coupling member 93 moves in a direction F intersecting the central axis C, and moves to a position on distally side than the main body 80.

The lesioned mucous membrane portion P6 supported by the coupling member 93 is ablated (elevated) from the submucosal layer P7. Thereafter, the high-frequency knife W2 within the second channel 12 is made to project to distally side than the attachment 3, and resects the lesioned mucous membrane portion P6.

If the lever 35 is operated to bring the shaft 52 into the retracted state, the bar 90 rotates about the pair of through-holes 81 as a center due to the elastic force of the spring member 100, and the coupling member 93 moves in a direction opposite to the intersecting direction F and returns to the position shown in FIG. 15.

The resected lesioned mucous membrane portion P6 is recovered by gripping forceps (not shown) inserted through the second channel 12.

The insertion part 10 of the endoscope 1 is pulled out from the patient's mouth, required treatment is performed, and a series of treatment is ended.

As described above, according to the endoscope system 2 according to the present embodiment, the first channel 11 of the endoscope 1 is sealed on the distal end side of the insertion part 10. Thus, the time and effort for cleaning the inside of the first channel 11 of the endoscope 1 after use in a procedure is reduced.

When the shaft 52 is brought into the projected state from the retracted state, since the coupling member 93 of the bar 90 moves in the direction F intersecting the central axis C, the lesioned mucous membrane portion P6 supported by the coupling member 93 can be ablated from the submucosal layer P7.

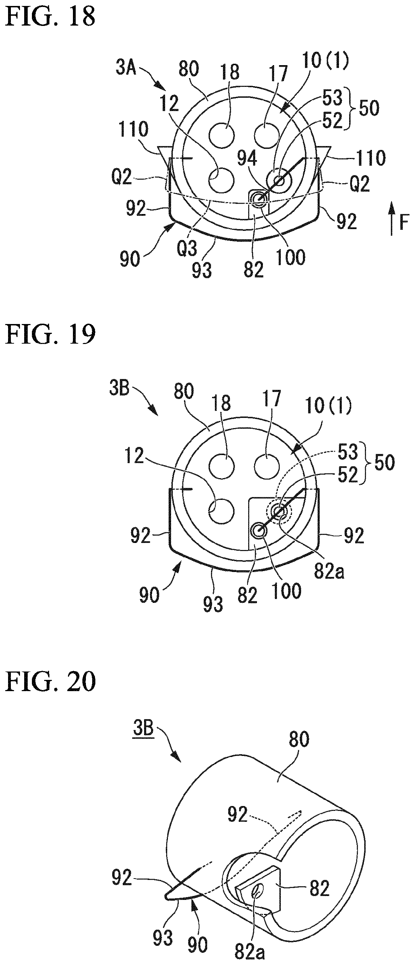

FIG. 17 is a perspective view of an attachment of a modified example of the present embodiment. FIG. 18 is a front view of a distal end part when the attachment of FIG. 17 is attached to the insertion part of the endoscope. In the present embodiment, as in an attachment 3A shown in FIGS. 17 and 18, a pair of projections 110 provided on the outer peripheral surface of the main body 80 may be included instead of the supporting member 82 and the spring member 100 of the attachment 3.

The respective projections 110 are formed so that an interval between portions of respective arm members 92 coming into contact with the respective projections increases as the coupling member 93 moves in the aforementioned intersecting direction F. That is, the width of the pair of projections 110 as a whole is larger (longer) than the distance between the pair of arm members 92 in a natural state.

If the attachment 3A configured in this way is attached to the insertion part 10 and the shaft 52 is brought from the retracted state and to the projected state, the arm members 92 respectively come into contact with the projections 110, and are deformed elastically and move so that the distance between the pair of arm members 92 increases as shown by a position Q2 in FIG. 18. In this case, the coupling member 93 is also deformed elastically and moves as shown as a position Q3.

When the shaft 52 is brought into the retracted state, the bar 90 rotates with the pair of through-holes 81 as a center due to the elastic forces of the pair of arm members 92 and the coupling member 93, and the distance between the pair of arm members 92 returns to an original state shown in FIG. 17.

FIG. 19 is a front view of the distal end part of the endoscope system 2 of a modified example of the present embodiment. FIG. 20 is a perspective view of an attachment of the endoscope system of the modified example of the present embodiment is used by cutting away a part of the attachment. As in an attachment 3B shown in FIGS. 19 and 20, when the attachment 3B is attached to the insertion part 10, a through-hole 82a through which the shaft 52 of the projectable unit 50 is inserted may be formed in the supporting member 82.

If such a configuration is adopted, the position of the attachment 3B in the circumferential direction with respect to the insertion part 10 can be easily specified by allowing the shaft 52 to be inserted through the through-hole 82a of the supporting member 82. That is, the through-hole 82a functions as a positioning index of the attachment 3B in the circumferential direction. In this case, since this positioning index is not provided on an outer surface in a state where the attachment 3B is attached to the insertion part 10, the insertability of the insertion part 10 can be maintained.

Although the first and second embodiments of the invention have been described above in detail with reference to the drawings, a specific configuration is not limited to the embodiments, and changes, combinations, and eliminations of the configuration are also included without departing from the scope of the invention. Moreover, it is obvious that the respective configurations shown in the respective embodiments may be appropriately combined and used.

For example, although an example in which the outer surface of the insertion part 10 is the distal end surface 14a of the distal end rigid part 14 has been shown in the first embodiment and the second embodiment, the outer surface may be a side surface of the insertion part 10.

Although an example in which the insertion part 10 of the endoscope 1 is provided with the bending part 15 that is bendably operated has been shown, the insertion part 10 may configure so that the bending part 15 is not provided.

Although the flexible endoscope 1 has been shown, a so-called rigid endoscope with a rigid insertion part may be adopted. In this case, the transmission performance of a force can be improved by using a rod-shaped member having no flexibility as a shaft-like member.

While the respective embodiments of the invention have been described above, the technical scope of the invention is not limited to the above embodiments. Combinations of constituent elements in the respective embodiments can be changed, various alternations can be added to the respective constituent elements, or omissions can be made, without departing from the concept of the invention. The invention is not to be considered as being limited by the foregoing description, and is limited only by the scope of the appended claims.

### Industrial Applicability

It is possible to provide the endoscope and the endoscope system in which the time and effort for cleaning the inside of a channel after being used in a procedure is reduced.

### Reference Signs List

- 1, 1A, 1B:: ENDOSCOPE
- 2:: ENDOSCOPE SYSTEM
- 3, 3A, 3B:: ATTACHMENT (CAP)
- 10:: INSERTION PART
- 14a:: DISTAL END SURFACE (OUTER SURFACE)
- 30:: OPERATION PART
- 40:: DRIVING WIRE (SHAFT-LIKE MEMBER)
- 50:: PROJECTABLE UNIT
- 51, 61:: FIXING PART
- 52:: SHAFT (MOVABLE PART)
- 53, 62:: COUPLING PART
- 66, 67:: LARGER-DIAMETER PART
- 66a, 67a:: RECESS
- 73:: DISTAL-END-SIDE RESTRICTING PART
- 74:: PROXIMAL-END-SIDE RESTRICTING PART
- 80:: MAIN BODY
- 90:: BAR (MOVABLE MEMBER)
- F:: INTERSECTING DIRECTION

## Claims

1. An endoscope comprising:
an insertion part;
an operation part attached to a proximal end part of the insertion part;
a shaft-like member inserted into the insertion part, a proximal end part of the shaft-like member extending to the operation part, and the shaft-like member being configured to be capable of advancing and retracting with respect to the insertion part; and
a projectable unit provided at a distal end part of the insertion part;
wherein the projectable unit includes:
a fixing part watertightly attached on an outer surface of the insertion part;
a movable part provided in a state where a distal end is exposed to an outside, and is movable in a direction intersecting the outer surface by advancing and retracting the shaft-like member; and
a coupling part which is elastically deformable and watertightly couples together the fixing part and the movable part.

2. The endoscope according to Claim 1, further comprising:
a distal-end-side restricting part restricting a range where the shaft-like member moves to a distal end side with respect to the insertion part.

3. The endoscope according to Claim 1, further comprising:
a proximal-end-side restricting part restricting a range where the shaft-like member moves to a proximal side with respect to the insertion part.

4. The endoscope according to Claim 1,
wherein the shaft-like member and the movable part are separated from each other when the shaft-like member moves to a proximal side, and
wherein the movable part is configured to be pushed to a distal end side by the shaft-like member when the shaft-like member moves to the distal end side.

5. The endoscope according to Claim 4,
wherein a proximal end part of the movable part is formed so as to project toward proximally than the coupling part, one of a proximal end part of the movable part and a distal end of the shaft-like member is provided with a connecting part, and the other of the proximal end part of the movable part and the distal end of the shaft-like member is provided with a larger-diameter part that is larger than the external diameter of the connecting part.

6. The endoscope according to Claim 5,
wherein a recess that is recessed toward a direction away from the connecting part is formed in a surface of the larger-diameter part that faces the connecting part.

7. The endoscope according to Claim 1,
wherein the shaft-like member and the movable part are coupled together.

8. An endoscope system comprising:
the endoscope according to Claim 1; and
a cap that is attachable to and detachable from an insertion part distal end that is an outer surface of the insertion part of the endoscope;
wherein the cap includes:
a main body that is formed in a tubular shape, a proximal end part of the main body attached to the insertion part; and
a movable member that is movably attached to the main body and that moves in a direction intersecting a central axis of the main body, and moves to a position distally side than the main body, when being moved from a state where the main body is attached to the insertion part and the movable part is moved from the insertion part distal end to the proximal end side, to a state where the movable part is moved from the insertion part distal end to the distal side.
